# EUROPEAN PATENT SPECIFICATION

(11) **EP 2 194 958 B1**
(45) Date of publication and mention of the grant of the patent: **29.02.2012**
(21) Application number: 08828587.9
(22) Date of filing: 02.09.2008
(51) Int. Cl.: A61K 8/35, A61K 8/37, A61Q 19/04

(54) **SUNLESS TANNING COMPOSITION WITH PHOTOSTABILIZED SUNSCREEN**
SELBSTBRÄUNUNGSZUSAMMENSETZUNG MIT LICHTSTABILISIERTEM SONNENSCHUTZ
COMPOSITION AUTOBRONZANTE AVEC ECRAN SOLAIRE PHOTOSTABILISE

(30) Priority: 31.08.2007 US 967084 P
(43) Date of publication of application: 16.06.2010
(73) Proprietor: PLAYTEX PRODUCTS, INC., Westport, CT 06880 (US)
(72) Inventor: SPAULDING, Laura A., Wayne, New Jersey 07470 (US); SANOGUEIRA, James, Wesley Hills, New York 10901 (US); DONOVAN, Barbara, Wayne, New Jersey 07470 (US)
(74) Representative: Berglund, Stefan
(86) International application number: PCT/US2008/075014
(87) International publication number: WO 2009/029931

(56) References cited:
- US-A1- 2004 057 912
- US-A1- 2004 247 536
- US-A1- 2006 257 338

## Description

### Cross Reference to Related Application

The present application claims the benefit of U.S. Provisional Patent Application Serial No. 60/967,084, filed August 31, 2007, entitled "Sunless Tanning Composition With Sunscreen" .

### Technical Field

The present invention generally relates to compositions for protecting the skin from overexposure to ultraviolet radiation and is, more particularly, directed to compositions that combine sunless tanning and sunscreen properties.

### Background of the Invention

Sunless tanning products have become increasingly popular and are often used in lieu of obtaining a tan in the traditional manner by sunbathing. Often, people will use a sunless tanning product prior to sunbathing to give them the appearance of having a tan so that they look more appealing in a bathing suit. However, known sunless tanning products do not typically provide protection against the harmful effects associated with prolonged exposure to the sun's radiation. As such, in order to minimize the sun's detrimental effects and avoid being burned by the sun, it is necessary to use a sunscreen product.

Overexposure to the sun's ultraviolet radiation is what causes sunburn. Ultraviolet radiation has a wavelength of about 290 nanometers (nm) to about 400 nm. Ultraviolet radiation over 320 nm to 400 nm is known as UV-A radiation and is primarily responsible for causing a tanned appearance upon sufficient exposure of skin thereto. Ultraviolet radiation in the 290 nm to 320 nm range is known as UV-B radiation and after sufficient exposure thereto can cause erythema of the skin, commonly referred to as sunburn. Overexposure to UV-A radiation can also cause sunburn. Continued skin damage resulting from unprotected exposure to ultraviolet radiation can cause more serious conditions such as, for example, skin cancer. Depending on weather conditions, even casual unprotected exposure to the sun's ultraviolet radiation can be detrimental to one's skin. Accordingly, it is generally recommended that a sunscreen product be applied to the skin before exposure to ultraviolet radiation.

Historically, where a sunless tanning product has been used, the user must still apply a sunscreen product to minimize exposure to ultraviolet radiation. A difficulty that can occur results from the interaction of the sunless tanning product and the sunscreen product. In general, the sunscreen's effectiveness is diminished by the interaction. Moreover, people would prefer to minimize the number of different materials they apply to their skin. To address these problems, efforts have been made to incorporate sunless tanning compositions with sunscreen products. These combination compositions generally use dihydroxyacetone (DHA) or erythrulose as the sunless tanning agent and avobenzone as the sunscreen agent. A problem that has occurred is that avobenzone is not photostable when used in combination with DHA or erythrulose. This instability causes the sunless tanning/sunscreen composition to significantly degrade and often become useless with respect to protection against ultraviolet radiation.

Based on the foregoing, it is an object of the present invention to provide a combination sunless tanning/sunscreen composition that improves upon conventional compositions and overcomes drawbacks and problems associated with the prior art.

### Summary of the Invention

In one aspect, the present invention resides in a cosmetic composition that includes a sunless tanning agent and a sunscreen agent. A photostabilizer is added to stabilize the sunscreen agent from the historically destabilizing effect of the sunless tanning agent. In this manner, UV protection is provided to the user when the cosmetic composition is applied to skin.

In another aspect, the present invention resides in a water-based sunless tanning/sunscreen composition comprising a sunless tanning agent, a sunscreen agent, and a photostabilizer. The sunless tanning agent is DHA, and the sunscreen is avobenzone. Because the avobenzone degrades when exposed to UV radiation when used with DHA, the photostabilizer is added to enable the avobenzone to retain most of its original ability to absorb the UV radiation. The photostabilizer is a low molecular weight copolymer of adipic acid and neopentyl glycol.

An advantage of the present invention is that through application of a single composition, a sunless tanning and sunscreen effect is achieved.

Another advantage of the present invention is that a combination sunless tanning and sunscreen composition is provided such that the effectiveness of the sunscreen agent does not appreciably degrade in the presence of the sunless tanning composition.

### Brief Description of the Drawings

Figure 1 is a graphical representation illustrating the photostability of a sunscreen composition having DHA and a photostabilizer, according to the present invention.
Figure 2 is a graphical representation illustrating the photostability of a sunscreen composition having DHA and a photostabilizer, according to the present invention.
Figure 3 is a graphical representation illustrating the photostability of a sunscreen composition having no DHA and a photostabilizer.
Figure 4 is a graphical representation illustrating the photostability of a sunscreen composition having DHA and no photostabilizer.
Figure 5 is a graphical representation of the effect of adipic acid and neopentyl glycol on the photostabilization of avobenzone in the presence of DHA.

### Detailed Description of the Preferred Embodiment

The present invention is a cosmetic composition applicable to the skin that provides a sunless tanning and sunscreen effect. In one embodiment, the composition is a water-based emulsion that includes one or more sunless tanning agents, one or more sunscreen agents capable of filtering, absorbing, and/or blocking ultraviolet (UV) radiation, and one or more photostabilizers. The composition may further include one or more skin conditioners (e.g., emollients, humectants, and the like), film forming agents, emulsifying agents, thickeners, preservatives, pH adjusters, coloring agents, and fragrances. The composition may be in the form of, but is not limited to, a lotion, spray, gel, cream, foam, or like topical products.

The sunless tanning agent, when applied to human skin, imparts pigment to darken the skin to give the appearance of a suntan without exposing the skin to the sun or other radiant sources of energy (e.g., those found in tanning beds or UV lamps). Materials that can be used as sunless tanning agents include, but are not limited to, dihydroxyacetone (DHA), erythrulose, pigments such as melanin, botanical extracts of various plants (e.g., mahakanni (eclipta alba)), and any combinations of the foregoing. DHA is a simple carbohydrate derived from plant sources and glycerin. When used as a tanning agent, DHA reacts with proteins in the skin to produce a tanning effect over a period of time. Erythrulose, which is chemically similar to DHA, is a natural sugar that reacts with amino acids in keratin protein found in dead skin cells to produce a tanning effect.

In the present invention, the sunless tanning agent is DHA and is present in an amount of 5.0 wt. %.

The sunscreen agent forming part of the sunless tanning/sunscreen composition preferably absorbs, filters, and/or blocks both UV-A radiation as well as UV-B radiation. In the present invention, the sunscreen agents include avobenzone, octocrylene, octisalate, homosalate, and combinations of the foregoing.

A photostabilizer is included in the sunless tanning/sunscreen composition of the present invention. The photostabilizer enables a photoactive compound (such as avobenzone) to retain at least a portion of its original ability to absorb, filter, and/or block irradiation at a particular wavelength (or over a range of wavelengths). Avobenzone is not photostable in the presence of a sunless tanning agent (DHA in one embodiment). In general, avobenzone absorbs ultraviolet radiation in an approximate range of about 350-370 nm. The use of a suitable photostabilizer in the sunless tanning/sunscreen composition of the present invention causes the avobenzone to maintain a substantial portion of ultraviolet radiation absorbance over the above-described range. In one embodiment of the present invention, a "substantial portion" of the absorbance of the avobenzone should be construed to mean at least about 78%. In another embodiment of the present invention, a "substantial portion" of the absorbance of the avobenzone should be construed to mean at least about 92%.

The photostabilizer that is used with avobenzone in the presence of DHA is a low molecular weight copolymer of adipic acid and neopentyl glycol (available under the trade name POLYCRYLENE from RTD Hallstar of Hackettstown, New Jersey).

The amount of acid/neopentyl glycol copolymer as a photostabilizer is from of 1.0 wt.%. or 2.0 wt%.

One or more skin conditioners may also be included in the sunless tanning/sunscreen composition of the present invention. Skin conditioners that may be used include, but are not limited to, emollients, humectants, refatting agents, and the like.

Emollients soften the skin surface and also control a rate of evaporation of the sunless tanning/sunscreen composition from the skin surface. Suitable emollients include, but are not limited to, cocoglycerides, cyclomethicone, dimethicone, dicapryl maleate, caprylic/capric triglyceride, isopropyl myristate, octyl stearate, isostearyl linoleate, lanolin oil, coconut oil, cocoa butter, shea butter, olive oil, avocado oil, aloe extracts, jojoba oil, castor oil, fatty acids such as oleic acid and stearic acid, fatty alcohols such as cetyl alcohol and hexadecyl alcohol, diisopropyl adipate, hydroxybenzoate ester, benzoic acid esters of C₉-C₁₅ alcohols, isononyl iso-nonanoate, alkanes such as mineral oil, silicones such as dimethyl polysiloxane, ether such as polyoxypropylene butyl ether and polyoxypropylene cetyl ether, C₁₂-C₁₅ alkyl benzoate, and combinations thereof. In the composition of the present invention, the total amount of emollient is in a range of 0.25 wt. % to 30 wt. %. Preferably, the emollient is present in an amount 0.25 wt.% to 4 wt.%.

Humecants can be included in the sunless tanning/sunscreen composition of the present invention to stabilize the water content, promote water retention, and control evaporation. Suitable humectants include, but are not limited to, glycerin, pentylene glycol, hexylene glycol, propylene glycol, butylene glycol, sorbitol, PEG-4, and combinations thereof. A preferred humectant is glycerin, which when used in the sunless tanning/ sunscreen composition of the present invention is present in an amount from 1.0 wt.% to 10 wt. %, preferably in an amount from 2.0 wt. % to 6.0 wt. %, and more preferably in an amount from 3.0 wt. % to 5.0 wt. %.

Other skin conditioners that may be incorporated into the present invention are hydrophilic benzoate esters. One such skin conditioner is methyl gluceth-20 benzoate (available under the trade name FINSOLV EMG-20 from Finetex of Edison, New Jersey), which when used can function as an emollient, aid in solublizing the sunscreen component, and help to stabilize the composition. When used in the sunless tanning/sunscreen composition of the present invention, methyl gluceth-20 benzoate would be present in 0.25 wt. % to 4.0 wt. %. However, the present invention is not limited in this regard as amounts of methyl gluceth-20 benzoate different from those described above may be used in the sunless tanning/sunscreen composition of the present invention without departing from the broader aspects thereof.

Another skin conditioner that may be present in the sunless tanning/sunscreen composition of the present invention is caprylyl glycol (also known as 1,2-octanediol (available under the trade name LEXGARD O from Inolex Chemical Company of Philadelphia, Pennsylvania)). Caprylyl glycol conditions the skin by providing a refatting function. Still other skin conditioners that may be incorporated into the composition include, but are not limited to, petrolatum, glycerin, aloe vera, allantoin, sodium PCA, combinations of the foregoing with or without caprylyl glycol, and the like. In the composition of the present invention, skin conditioners may be present in amounts up to about 30 wt. %. Preferably, skin conditioners may be present in an amount between 0.1 wt.% and 10 wt.%, and more preferably between 0.25 wt.% and 5 wt.%.

A film forming agent may also be included in the composition of the present invention. Generally, the film forming agent is a hydrophobic material that provides a waterproofing effect to the composition when applied on skin. Suitable film forming agents include, but are not limited to, copolymers of acrylates or acrylates/acrylamides, combinations of acrylates and C₁₂-C₂₂ alkylmethacrylate copolymers, polyethylenes, waxes, esters of polyvinyl pyrrolidone (PVP)/dimethiconylacrylate/polycarbamylpolyglycol, butylated PVP, PVP/hexadecene copolymer, PVP/eicosene copolymer, tricontanyl PVP, combinations of the foregoing, and the like. Preferred film forming agents are polyester-7 (and) neopentyl glycol diheptanoate (available under the trade name LEXFILM SUN from Inolex Chemical Company of Philadelphia, Pennsylvania). In the composition of the present invention, film forming agents can be present in amounts from 0.1 wt. % to 5.0 wt. % and preferably from 1.0 wt. % to 3.0 wt. %. However, the present invention is not limited in this regard as other film forming agents known to those skilled in the pertinent art to which the present invention pertains and different amounts of film forming agents may be used in the sunless tanning/sunscreen components of the present invention.

Various primary and secondary emulsifying agents may be included in the sunless tanning/sunscreen composition of the present invention to provide suitable rheological characteristics to the sunless tanning/sunscreen composition. Primary emulsifying agents that may be used include, but are not limited to, acrylate crosspolymers, polyacrylic acid, sodium methacrylate, sodium polyacrylate, polyacrylates, and combinations thereof. Primary emulsifying agents preferably include stearic acid, polysorbates such as polyoxyethylene (20) sorbitan monostearate (available under the trade name TWEEN 60 from Sigma-Aldrich Corporation of Milwaukee, Wisconsin), stearyl ethers such as polyoxyethylene (2) stearyl ether (available under the trade name BRIJ 72 from Sigma-Aldrich Corporation of Milwaukee, Wisconsin) and polyoxyethylene (20) stearyl ether (available under the trade name BRU 78 from Sigma-Aldrich Corporation of Milwaukee, Wisconsin), C₁₄-C₂₂ alcohol (and) C₁₂-C₂₀ alkyl glucoside (available under the trade name MONTANOV L from Adinop Co., Ltd. of Bangkok, Thailand), combinations of the foregoing, and the like. In the present invention, the amount of primary emulsifying agent is preferably from about 0.01 wt. % to about 10 wt. % and more preferably from about 0.1 wt. % to about 5.0 wt. %.

Secondary emulsifying agents that when used in conjunction with the primary emulsifying agents provide synergistic effects may also be incorporated into the sunless tanning/sunscreen composition of the present invention. Such secondary emulsifying agents include, but are not limited to, cetyl alcohol, stearyl alcohol, combinations of the foregoing, and the like. When used, the amount of secondary emulsifying agent in the composition of the present invention is preferably from 0.01 wt. % to 5.0 wt. %, more preferably from 0.05 wt. % to 2.0 wt. %, and still more preferably from 0.1 wt. % to 0.5 wt. %. However, the present invention is not limited in this regard as amounts other than those specified above can also be employed.

Thickeners may also be used in the sunless tanning/sunscreen composition of the present invention and can include synthetic and natural gum or polymer products, polysaccharide thickening agents, associative thickeners, anionic associative rheology modifiers, nonionic associative rheology modifiers, oil-thickening agents, acrylates/C₁₀-C₃₀ alkylacrylate crosspolymer, acrylates/aminoacrylates/C₁₀-C₃₀ alkyl PEG-20 itaconate copolymer, acrylates copolymer, acrylates/steareth-20 methacrylate copolymer, acrylates/beheneth-25 methacrylate copolymer, PEG-150/decyl alcohol/SMDI copolymer, PVP, carbomer, PEG crosspolymer, acrylates/palmeth-25 acrylates copolymer, polysaccharides, polyacrylates, polyether-1, sodium magnesium silicates, sodium carbomers, sodium polyacrylates, sodium polymethacrylates, sodium polyacryloyldimethyl taurates, sodium acryloyldimethyl taurate copolymers, sodium carragenan, sodium carboxymethyl dextran, hydroxyethylcellulose, hydroxypropyl cyclodextran, bentonites, trihydroxystearin, aluminum-magnesium hydroxide stearate, xanthan gum, and any combinations thereof. Preferably, the thickening agent is carbomer, sodium carbomer, xanthan gum, or any combinations thereof. The amount of thickener when used in the sunless tanning/sunscreen composition of the present invention is from 0.01 wt. % to 10 wt. % and preferably from 0.1 wt. % to 5.0 wt. %.

In one embodiment, the sunless tanning/sunscreen composition of the present invention includes a copolymer of hydroxyethyl acrylate and sodium acryloylmethyl taurate (available under the trade name SEPINOV EMT 10 from Adinop Co., Ltd. of Bangkok, Thailand). This copolymer provides both emulsifying properties and thickening properties in water-based compositions. It also imparts a characteristic texture to the composition that allows the composition to be applied with a creamy, smooth tactile sensation. When used, the amount of this copolymer is preferably from 0.01 wt. % to 10 wt. % and more preferably from 0.1 wt. % to 5.0 wt. %. However, the present invention is not limited in this regard as amounts of hydroxyethyl acrylate and sodium acryloylmethyl taurate other than those set forth above can also be used.

Materials useful in adjusting the pH of the sunless tanning/sunscreen composition of the present invention may also be included. Such materials include, but are not limited to, sodium hydroxide, triethanolamine, salts of EDTA, and citric acid. Preferably, the composition is adjusted to a pH from 4.0 to 4.5 using a suitable amount of citric acid.

Preservatives may also be included in the composition of the present invention to protect the composition from microbial contamination and/or oxidation. Preservatives that may be incorporated into the composition include, but are not limited to, diazolidinyl urea, iodopropynyl butylcarbamate, chloromethylisotiazolinone, methylisothiazolinone, vitamin E and its derivatives including vitamin E acetate, vitamin C, butylated hydroxytoluene (BHT), butylparaben, ethylparaben, methylparaben, propylparaben, isobutylparaben, phenoxyethanol, and combinations thereof. The amount of preservative present in the sunless tanning/sunscreen composition of the present invention is preferably from 0.01 wt. % to 2 wt. %. However, the present invention is not limited in this regard as other weight percentages can also be employed.

The sunless tanning/sunscreen composition of the present invention may also include at least one coloring agent. Coloring agents include, but are not limited to, caramel, melanin, extracts from various botanicals, oxides of iron, zinc, and/or titanium, dyes, combinations of the foregoing, and the like. One preferred coloring agent is caramel. When used, the amount of caramel in the sunless tanning/sunscreen composition of the present invention is from 0.1 wt. % to 1.0 wt. %. However, the present invention is not limited in this regard as other weight percentages can also be employed.

Fragrances may also be included in the sunless tanning/sunscreen composition of the present invention. Fragrances are generally aromatic compounds that impart aesthetically pleasing qualities of smell. Materials that can be used to provide fragrance to the sunless tanning/sunscreen composition of the present invention include, but are not limited to, essential oils, extracts of certain flowers (e.g., rose, jasmine, and the like), extracts of certain fruits (e.g., coconut, apple, melon, and the like), alcohols, combinations of the foregoing, and the like. The composition of the present invention typically includes up to 1.0 wt.% fragrance and preferably from 0.05 wt. % to 0.5 wt. %. However, the present invention is not limited in this regard as other weight percentages can also be employed.

### Example 1 - Photostability of compositions having at least one of a sunless tanning agent and a photostabilizing agent

Table 1 below provides formulations for compositions having at least one of a sunless tanning agent and a photostabilizing agent. All amounts are in weight percents (wt.%) based on the total weight of the composition.

**Table 1**

| Chemical Name | Trade Name | Sample 1 | Sample 2 | Sample 3 | Sample 4 |
|---|---|---|---|---|---|
| DHA (sunless tanning agent) | | 5.0 | 5.0 | -- | 5.0 |
| Adipic acid and neopentyl | Polycrylene | 1.0 | 2.0 | 1.0 | -- |
| glycol copolymer (photostabilizer) | | | | | |
| Avobenzone (sunscreen agent) | | 3.0 | 3.0 | 3.0 | 3.0 |
| Homosalate | | 5.0 | 5.0 | 5.0 | 5.0 |
| Octisalate | | 5.0 | 5.0 | 5.0 | 5.0 |
| Octocrylene | | 2.4 | 2.4 | 2.4 | 2.4 |
| Other components according to present invention | | Q.S. to 100 wt.% | | | |

Each of the Samples in Table 1 was analyzed for photostability. The absorbance of each Sample was measured using a Labsphere UV-1000S Ultraviolet Transmittance Analyzer (available from Labsphere of North Sutton, New Hampshire) before and after 30 joules per centimeter squared (J/cm²), 40 J/cm², 50 J/cm², and 100 J/cm² irradiation with a solar simulator. The solar simulator used was a Model 16S Solar Simulator (available from Solar Light Company of Glenside, Pennsylvania) equipped with a WG 320 filter to transmit radiation greater than 290 nm. The output from the solar simulator was monitored using a PMA 2105 UV-B DCS Detector (available from Solar Light Company of Glenside, Pennsylvania) (biologically weighted) and controlled by a PMA 2100 Automatic Dose Controller (also available from Solar Light Company of Glenside, Pennsylvania).

A substrate used for testing the compositions of each of the Samples comprised sandblasted plates (available from Schonberg GmbH & Co.). These plates measured about 5.0 millimeters (mm) square by about 0.25 nun thick. Upon having DHA applied thereto, the plates (the substrates) remained unreactive after long term exposure to UV radiation.

Slides for testing were prepared by drawing the composition of each Sample into a pipette and uniformly applying the composition to the substrate with an application dose corresponding to 2 milligrams per centimeter squared (mg/cm²). The composition was then spread by finger first in a circular motion then in a side-to-side motion. The substrate was then allowed to dry for 15 to 20 minutes.

To test photostability, the prepared slide was positioned on the Ultraviolet Transmittance Analyzer, and a scan of a one square centimeter spot was made. The slide was then transferred to a holder adjacent the solar simulator and, using calipers, positioned such that the beam of UV radiation exiting the solar simulator illuminated the same one square centimeter spot. The intensity of the illumination was varied in accordance with various settings defined by the software of the solar simulator, namely, (a) UV-B 290-320 nm; (b) UV-A 320-400 nm; (c) SPF 290-400 nm; (d) Spectral Irradiance, Noon, July 3, Albuquerque, New Mexico; and (e) SPF Spectral Irradiance and Erythemal Effectiveness.

Following a pretreatment exposure of 5 J/cm² radiation, each slide was again placed in position on the Ultraviolet Transmittance Analyzer, and a scan of the exposed spot was made. The procedure was repeated on the same spot on the slide and exposed to 30 J/cm² radiation.

Table 2 below provides photostability data for each of the Samples listed above in Table 1.

**Table 2**

| | Photostability (%) at 310 nm | | | | | Photostability (%) at 370 nm | | | | |
|---|---|---|---|---|---|---|---|---|---|---|
| Sample | 5J/cm² * | 30 J/cm² | 40 J/cm² | 50 J/cm² | 100 J/cm² | 5 J/cm² * | 30 J/cm² | 40 J/cm² | 50 J/cm² | 100 J/cm² |
| Sample 1 | 100 | 99.2 | 99.6 | 100 | 100 | 100 | 98.6 | 94.5 | 88.8 | 78.4 |
| Sample 2 | 100 | 97.9 | 99.5 | 98.5 | 95.0 | 100 | 98.5 | 96.3 | 97.4 | 92.3 |
| Sample 3 | 100 | 99.3 | 98.6 | 98.8 | 94.8 | 100 | 98.7 | 98.1 | 94.8 | 92.2 |
| Sample 4 | 100 | 100 | 99.3 | 100 | 100 | 100 | 91.4 | 84.0 | 78.5 | 49.3 |

| | | | | | | | | | | |
|---|---|---|---|---|---|---|---|---|---|---|
| * Pre-irradiation baseline | | | | | | | | | | |

As can be seen from the above data, Samples 1 and 2 according to the present invention with DHA and photostabilizer at 370 nm showed a marked increase in photostability at the higher irradiation levels, especially when compared to Sample 4 (comparative example outside of the present invention). Most notably, Sample 2 demonstrates comparable photostability to Sample 3 at 100 J/cm² and about an 87% increase in photostability over Sample 4 at 100 J/cm², further exemplifying the unexpected photostability achieved by the present invention.

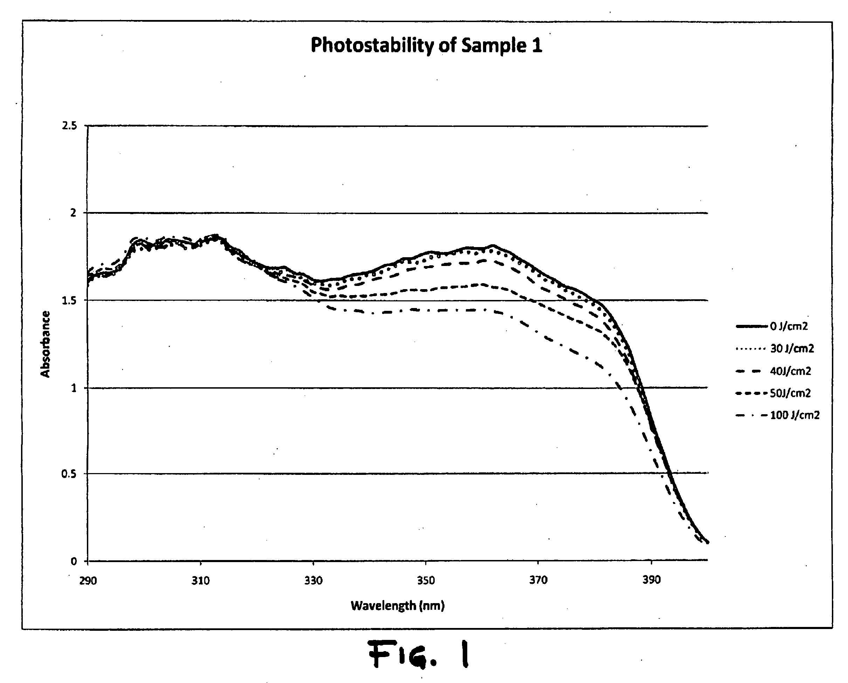

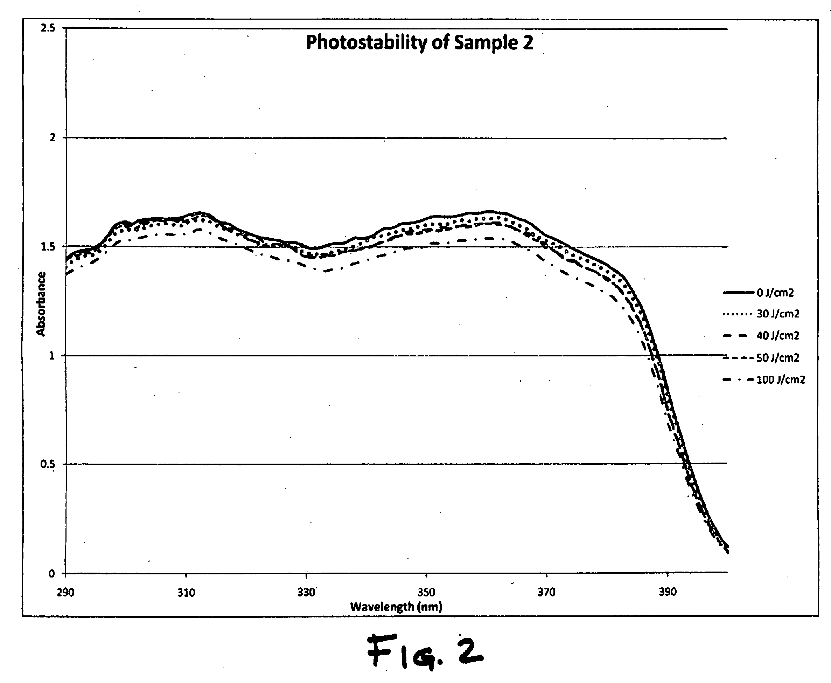

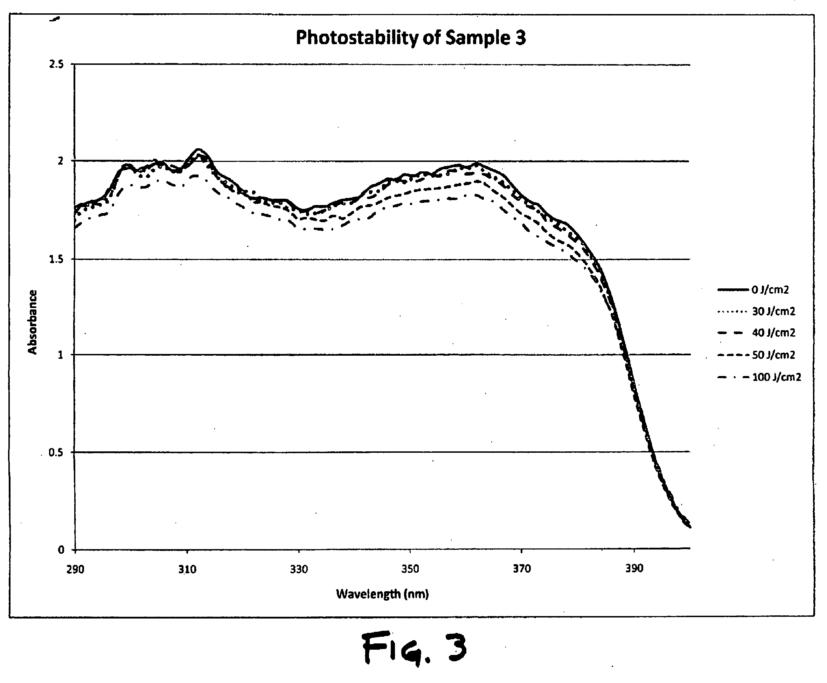

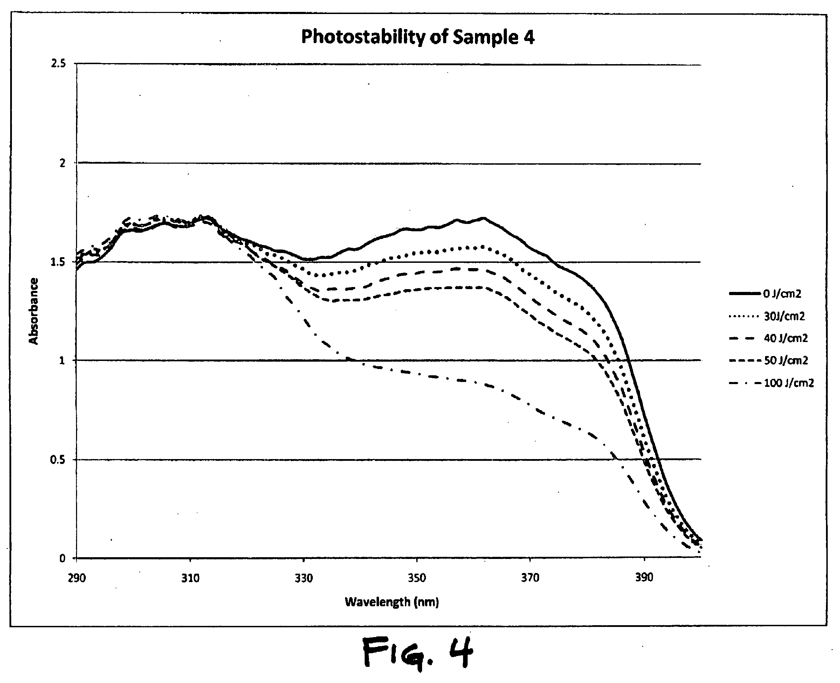

Referring to Figures 1 through 4, curves illustrating the photostability (absorbance versus wavelength) for each Sample are shown and provide a visual representation of a portion of the data shown in Table 2 above.

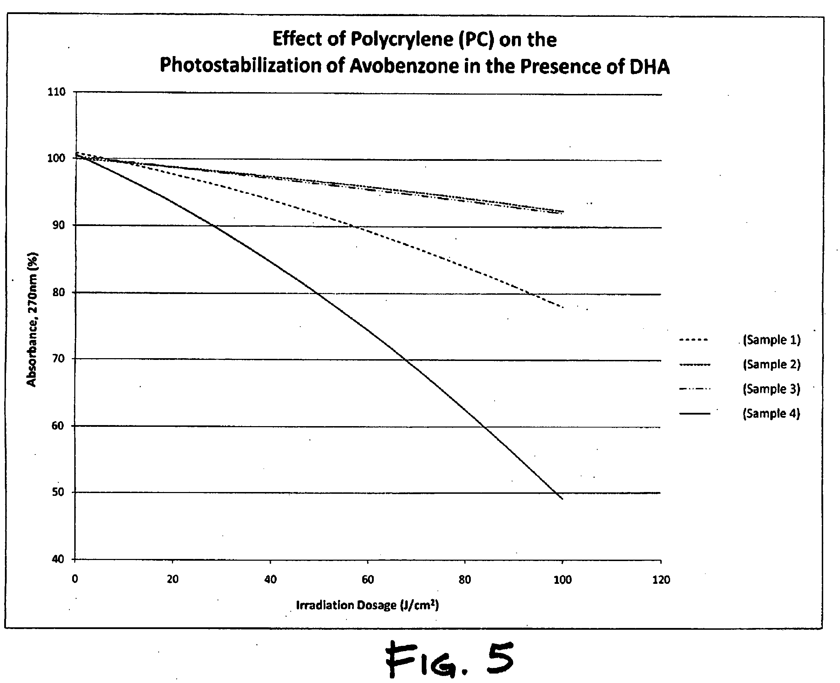

Referring now to Figure 5, the effect of the adipic acid and neopentyl glycol copolymer (POLYCRYLENE) on the photo stabilization of avobenzone in the presence of DHA as the sunless tanning agent is shown. As can be seen from the graph, Samples 1 and 2, according to the present invention, each having 5% DHA and 1% and 2% photostabilizer, respectively, maintain a high level of absorbance as the irradiation dosage is increased.

Although this invention has been shown and described with respect to the detailed embodiments thereof, it will be understood by those of skill in the art that various changes may be made and equivalents may be substituted for elements thereof without departing from the scope of the invention. In addition, modifications may be made to adapt a particular situation or material to the teachings of the invention without departing from the essential scope thereof. Therefore, it is intended that the invention not be limited to the particular embodiments disclosed in the above detailed description, but that the invention will include all embodiments falling within the scope of the appended claims.

## Claims

1. A cosmetic composition, comprising:
a sunless tanning agent selected from the group consisting of DHA, in an amount of 5.0 wt. %;
a sunscreen agent comprising avobenzone in an amount of 3.0 wt. %, having a characteristic UV radiation absorbance, and 5.0 wt. % homosalate, 5.0 wt. % octisalate, 2.4 wt. % octocrylene; and
a photostabilizer that stabilizes said sunscreen agent in the presence of said sunless tanning agent to allow said sunscreen agent to maintain a portion of said characteristic UV radiation absorbance thereof over a range of UV radiation, thereby allowing said sunscreen agent to remain photostable when subjected to said UV radiation, wherein said photostabilizer is selected from the group consisting of low molecular weight copolymers of adipic acid and neopentyl glycol, and wherein said photostabilizer is present in an amount of 1.0 wt. % or 2.0 wt. %.

2. The cosmetic composition of claim 1, further comprising at least one of an emollient, a humectant, a film forming agent, an emulsifying agent, a thickener, a preservative, a coloring agent, and a fragrance.

3. The cosmetic composition of claim 2, wherein said emollient is selected from the group consisting of cocoglycerides, cyclomethicone, dimethicone, dicapryl maleate, caprylic/capric triglyceride, isopropyl myristate, octyl stearate, isostearyl linoleate, lanolin oil, coconut oil, cocoa butter, shea butter, olive oil, avocado oil, aloe extracts, jojoba oil, castor oil, fatty acids, fatty alcohols, diisopropyl adipate, hydroxybenzoate ester, benzoic acid esters of C₉-C₁₅ alcohols, isononyl iso-nonanoate, alkanes, silicones, ethers, C₁₂-C₁₅ alkyl benzoate, and combinations thereof

4. The cosmetic composition of claim 3, wherein said emollient is present in said cosmetic composition in an amount of 0.25 wt. % to 4.0 wt. %.

5. The cosmetic composition of claim 2, wherein said humectant is selected from the group consisting of glycerin, pentylene glycol, hexylene glycol, propylene glycol, butylene glycol, sorbitol, PEG-4, and combinations thereof.

6. The cosmetic composition of claim 5, wherein said humectant is glycerin present in an amount of 1.0 wt. % to 10 wt. %.

7. The cosmetic composition of claim 2, wherein said film forming agent is selected from the group consisting of copolymers of acrylates, copolymers of acrylates/acrylamides, combinations of acrylates and C₁₂-C₂₂ alkylmethacrylate copolymers, polyethylenes, waxes, esters of polyvinyl pyrrolidone (PVP)/dimethiconylacrylate/polycarbamylpolyglycol, butylated PVP, PVP/hexadecene copolymer, PVP/eicosene copolymer, tricontanyl PVP, polyester-7 (and) neopentyl glycol diheptanoate, and combinations of the foregoing.

8. The cosmetic composition of claim 7, wherein said film forming agent is present in an amount of 0.1 wt. % to 5.0 wt. %.

9. The cosmetic composition of claim 2, wherein said emulsifying agent is a first emulsifying agent selected from the group consisting of acrylate crosspolymers, polyacrylic acid, sodium methacrylate, sodium polyacrylate, polyacrylates, stearic acid, polysorbates, stearyl ethers, C₁₄-C₂₂ alcohol (and) C₁₂-C₂₀ alkyl glucoside, and combinations of the foregoing.

10. The cosmetic composition of claim 9, wherein said first emulsifying agent is present in an amount of 0.01 wt. % to 10 wt. %.

11. The cosmetic composition of claim 9, further comprising a second emulsifying agent selected from the group consisting of cetyl alcohol, stearyl alcohol, and combinations of the foregoing.

12. The cosmetic composition of claim 11, wherein said second emulsifying agent is present in an amount of 0.01 wt. % to 5.0 wt. %.

13. The cosmetic composition of claim 2, wherein said thickener is selected from the group consisting of synthetic and natural gum or polymer products, polysaccharide thickening agents, associative thickeners, anionic associative rheology modifiers, nonionic associative rheology modifiers, oil-thickening agents, acrylates/C₁₀-C₃₀ alkylacrylate crosspolymer, acrylates/aminoacrylates/C₁₀-C₃₀ alkyl PEG-20 itaconate copolymer, acrylates copolymer, acrylates/steareth-20 methacrylate copolymer, acrylates/beheneth-25 methacrylate copolymer, PEG-150/decyl alcohol/SMDI copolymer, PVP, carbomer, PEG crosspolymer, acrylates/palmeth-25 acrylates copolymer, polysaccharides, polyacrylates, polyether-1, sodium magnesium silicates, sodium carbomers, sodium polyacrylates, sodium polymethacrylates, sodium polyacryloyldimethyl taurates, sodium acryloyldimethyl taurate copolymers, sodium carragenan, sodium carboxymethyl dextran, hydroxyethylcellulose, hydroxypropyl cyclodextran, bentonites, trihydroxystearin, aluminum-magnesium hydroxide stearate, xanthan gum, and any combinations thereof.

14. The cosmetic composition of claim 13, wherein said thickener is present in an amount of 0.01 wt. % to 10 wt. %.

15. The cosmetic composition of claim 2, wherein said preservative is selected from the group consisting of diazolidinyl urea, iodopropynyl butylcarbamate, chloromethylisotiazolinone, methylisothiazolinone, vitamin E, derivatives of vitamin E, vitamin E acetate, vitamin C, BHT, butylparaben, ethylparaben, methylparaben, propylparaben, isobutylparaben, phenoxyethanol, and combinations thereof.

16. The cosmetic composition of claim 15, wherein said preservative is present in an amount of 0.01 wt. % to 2 wt. %.

17. The cosmetic composition of claim 2, wherein said coloring agent is selected from the group consisting of caramel, melanin, botanical extract, zinc oxide, iron oxide, titanium dioxide, and combinations of the foregoing.

18. The cosmetic composition of claim 1, further comprising a skin conditioner selected from the group consisting of caprylyl glycol, petrolatum, glycerin, aloe vera, allantoin, sodium PCA, and combinations of the foregoing.

19. The cosmetic composition of claim 18, wherein said skin conditioner is present in an amount of up to 30 wt. %, preferably in an amount of 0.1 wt. % to 10 wt. %, and more preferably in an amount of 0.25 wt. % to 5 wt. %.

20. The cosmetic composition of claim 1, wherein said portion of said characteristic UV radiation absorbance is at least 78%, preferably at least 92%.

21. The cosmetic composition of claim 1, wherein said range of UV radiation is 350 nm to 370 nm.

22. The cosmetic composition according to claim 1, wherein the composition comprises 5.0 wt. % DHA, 1.0 wt. % adipic acid and neopentyl glycol copolymer, 3.0 wt. % avobenzone, 5.0 wt. % homosalate, 5.0 wt.% octisalate, 2.4 wt. % octocrylene, and up to 100 wt.% other components.

23. The cosmetic composition according to claim 1, wherein the composition comprises 5.0 wt. % DHA, 2.0 wt. % adipic acid and neopentyl glycol copolymer, 3.0 wt. % avobenzone, 5.0 wt. % homosalate, 5.0 wt.% octisalate, 2.4 wt. % octocrylene, and up to 100 wt.% other components.

## Patentansprüche

1. Ein kosmetisches Mittel umfassend:
einen Wirkstoff zur sonnenlosen Bräunung ausgewählt aus einer Gruppe bestehend aus DHA mit einem Anteil von 5,0 Gew.%;
ein Sonnenschutzmittel, aufweisend Avobenzon, mit einem Anteil von 3,0 Gew.%, mit einer charakteristischen Absorbtion von UV-Strahlung, Homosalat mit einem Anteil von 5,0 Gew.%, Octisalat mit einem Anteil von 5,0 Gew.% und Octocrilen mit einem Anteil von 2,4 Gew.%; und
einen Photostabilisator, welcher das Sonnenschutzmittel im Beisein des Wirkstoffes zur sonnenlosen Bräunung stabilisiert, um dem Sonnenschutzmittel zu ermöglichen, einen Teil der charakteristischen Absorption der UV-Strahlung gegenüber einem großen Bereich an UV-Strahlen beizubehalten, und dem Sonnenschutzmittel es zu ermöglichen photostabil zu bleiben, wenn es der UV-Strahlung ausgesetzt wird,
wobei besagter Photostabilisator ausgewählt ist aus einer Gruppe bestehend aus Copolymeren mit niedrigem Molekulargewicht der Adipinsäure und Neopentylglykol und wobei der Anteil des besagten Photostabilisators bei 1,0 Gew.% oder 2,0 Gew.% liegt.

2. Kosmetisches Mittel nach Anspruch 1, **dadurch gekennzeichnet, dass** es weiterhin zumindest ein weichmachendes Mittel, ein Befeuchtungsmittel, ein filmbildendes Mittel, ein emulgierendes Mittel, ein Verdickungsmittel, ein Konservierungsmittel, ein Farbstoff und ein Duftstoff.

3. Kosmetisches Mittel nach Anspruch 2, **dadurch gekennzeichnet, dass** das weichmachendes Mittel ausgewählt ist aus einer Gruppe, bestehend aus Cocoglyceriden, Cyclomethicon, Dimethicon, Dicaprylmaleat, Capryl/Caprintriglycerid, Isopropylmyristat, Octylstearat, Isostearyllinoleat, Lanolinöl, Kokosöl, Kakaobutter, Sheabutter, Olivenöl, Avocadoöl, Aloeextrakt, Jojobaöl, Rizinusöl, Fettsäuren, Diisopropyladipat, Hydroxibenzoatester, Benzoesäureester von C₉-C₁₅ Alkoholen, Isononyl-iso-nanonate, Alkane, Silikone, Ether, C₁₂-C₁₅ Alkylbenzoate und Kombinationen daraus.

4. Kosmetisches Mittel nach Anspruch 3, **dadurch gekennzeichnet, dass** das weichmachende Mittel in der kosmetischen Zusammensetzung mit einem Anteil von 0,25 Gew.% bis 4,0 Gew.% vorhanden ist.

5. Kosmetisches Mittel nach Anspruch 2, **dadurch gekennzeichnet, dass** das Befeuchtungsmittel ausgewählt ist aus einer Gruppe bestehend aus Glycerin, Pentylenglycol, Hexylenglycol, Propylenglycol, Butylenglycol, Sorbitol, PEG-4 und Kombinationen daraus.

6. Kosmetisches Mittel nach Anspruch 5, **dadurch gekennzeichnet, dass** das Befeuchtungsmittel Glycerin ist, mit einem Anteil von 1,0 Gew.% bis 10 Gew.%.

7. Kosmetisches Mittel nach Anspruch 2, **dadurch gekennzeichnet, dass** das filmbildende Mittel ausgewählt ist aus einer Gruppe bestehend aus Copolymeren von Acrylaten, Copolymeren von Acrylaten/Acrylamiden, Kombinationen von Acrylaten und C₁₂-C₂₂ Alkylmethacrylatcopolymeren, Polyethylenen, Wachsen, Estern von Polyvinylpyrrolidon (PVP)/Dimethiconylacrylat/Polycarbamylpolyglycol, butyliertem PVP, PVP/Hexadecen Copolymer, PVP/Eicosen Copolymer, Tricontanyl PVP, Polyester-7 (und) Neopentyl Glycol Diheptanoat und Kombinationen daraus.

8. Kosmetisches Mittel nach Anspruch 7, **dadurch gekennzeichnet, dass** das filmbildendes Mittel in einem Anteil von 0,1 Gew.% bis 5,0 Gew.% vorhanden ist.

9. Kosmetisches Mittel nach Anspruch 2, **dadurch gekennzeichnet, dass** das emulgierende Mittel ein erstes emulgierende Mittel ist, ausgewählt aus einer Gruppe bestehend aus Acrylatkreuzpolymeren, Polyacrylsäure, Natriummethacrylat, Natriumpolyacrylat, Polyacrylaten, Stearinsäure, Polysorbaten, Stearylethern, C₁₄-C₂₂ Alkohol (und) C₁₂-C₂₀ Alkylglucosid und
Kombinationen daraus.

10. Kosmetisches Mittel nach Anspruch 9, **dadurch gekennzeichnet, dass** das erste emulgierende Mittel in einem Anteil von 0,01 Gew.% bis 10 Gew.% vorhanden ist.

11. Kosmetisches Mittel nach Anspruch 9, **dadurch gekennzeichnet, dass** es weiterhin ein zweites emulgierendes Mittel aufweist, ausgewählt aus einer Gruppe bestehend aus Cetylalkohol, Stearylalkohol und Kombinationen daraus.

12. Kosmetisches Mittel nach Anspruch 11, **dadurch gekennzeichnet, dass** das zweite emulgierende Mittel in einem Anteil von 0,01 Gew.% bis 5,0 Gew.% vorhanden ist.

13. Kosmetisches Mittel nach Anspruch 2, **dadurch gekennzeichnet, dass** das Verdickungsmittel ausgewählt ist aus einer Gruppe bestehend aus synthetischen oder natürlichen Gummi- oder Polymerprodukten, polysacchariden Verdickungsmittel, assoziativen Verdickungsmitteln, anionischen assoziativen Rheologiemodifizierern, nichtionischen assoziativen Rheotogiemodifizierern, Ölverdickungsmittel, Acrylate/Aminoacrylate/C₁₀₋₃₀ Alkyl PEG-20 Itaconat Copolymer, Acrylatcopolymer, Acrylat/Steareth-20 Methacrylatcopolymer, Acrylate/Beheneth-25 Methacrylatcopolymer, PEG-150/Decylalkohol/SMDI Copolymer, PVP, Carbomer, PEG-Kreuzpolymer, Acrylate/Palmeth-25 Acrylate Copolymer, Polysaccharide, Polyacrylate, Polyether-1, Natrium Magnesium Silicate, Natriumcarbomere, Natriumpolyacrylate, Natriumpolymethacrylate, Natriumpolyacryloyldimethyltaurate, Natriumacryloyldimethyltauratcopolymere, Natriumcarrageenan, Natriumcarboxymethyldextran, Hydroxyethylcellulose, Hydroxypropylcyclodextran, Bentonite, Trihydroxystearin, Aluminium-Magnesium Hydroxid, Stearat, Xanthangummi und Kombinationen daraus.

14. Kosmetisches Mittel nach Anspruch 13, **dadurch gekennzeichnet, dass** das Verdickungsmittel in einem Anteil von 0,01 Gew.% bis 10 Gew.% vorhanden ist.

15. Kosmetisches Mittel nach Anspruch 2, **dadurch gekennzeichnet, dass** das Konservierungsmittel ausgewählt ist aus einer Gruppe bestehend aus Diazolidinylharnstoff, Iodoprynylbutylcarbamat, Chlormethylisothiazolinon, Methylisothiazolinon, Vitamin E, Derivate von Vitamin E, Vitamin E Acetat, Vitamin C, BHT, Butylparaben, Ethylparaben, Methylparaben, Propylparaben, Isobutylparaben, Phenoxyethanol und Kombinationen daraus.

16. Kosmetisches Mittel nach Anspruch 15, **dadurch gekennzeichnet, dass** das Konservierungsmittel in einem Anteil von 0,01 Gew.% bis 2 Gew.% vorhanden ist.

17. Kosmetisches Mittel nach Anspruch 2, **dadurch gekennzeichnet, dass** der Farbstoff ausgewählt ist aus einer Gruppe bestehend aus Karamel, Melamin, botanischen Extract, Zinkoxid, Eisenoxid, Titandioxid und Kombinationen daraus.

18. Kosmetisches Mittel nach Anspruch 1, **dadurch gekennzeichnet, dass** es weiterhin einen Haut-Conditioner aufweist, ausgesucht aus einer Gruppe bestehend aus Caprylyl Glycol, Vaseline, Glycerin, Aloe Vera, Allantoin, Natrium-PCA und Kombinationen daraus.

19. Kosmetisches Mittel nach Anspruch 18, **dadurch gekennzeichnet, dass** der Haut-Conditioner in einem Anteil von bis zu 30 Gew.%, vorzugsweise in einem Anteil von 0,1 Gew.% bis 10 Gew.%,besonders bevorzugt in einem Anteil von 0,25 Gew.% bis 5 Gew.%, vorhanden ist.

20. Kosmetisches Mittel nach Anspruch 1, **dadurch gekennzeichnet, dass** der Teil der charakteristischen Absorption der UV-Strahlung zumindest 78%, vorzugsweise zumindest 92% beträgt.

21. Kosmetisches Mittel nach Anspruch 1, **dadurch gekennzeichnet, dass** der Bereich der UV Strahlung von 350 nm bis 370 nm reicht.

22. Kosmetisches Mittel nach Anspruch 1, **dadurch gekennzeichnet, dass** die Verbindung 5,0 Gew.% DHA, 1,0 Gew.% Adipinsäure- und Neopentylglycol-Copolymer, 3,0 Gew.% Avobenzon, 5,0 Gew.% Homosalat, 5,0 Gew.% Octisalat, 2,4 Gew.% Octocrilen und bis zu 100 Gew.% andere Komponenten aufweist.

23. Kosmetisches Mittel nach Anspruch 1, **dadurch gekennzeichnet, dass** die Verbindung 5,0 Gew.% DHA, 2,0 Gew.% Adipinsäure- und Neopentylglycol-Copolymer, 3,0 Gew.% Avobenzon, 5,0 Gew.% Homosalat, 5,0 Gew.% Octisalat, 2,4 Gew.% Octocrilen und bis zu 100 Gew.% andere Komponenten aufweist.

## Revendications

1. Composition cosmétique comprenant :
un agent autobronzant choisi dans le groupe constitué par la DHA, en une quantité de 5,0 % en poids ;
un agent d'écran solaire comprenant de l'avobenzone en une quantité de 3,0 % en poids, ayant une absorption de rayonnement d'UV caractéristique et 5,0 % en poids d'homosalate, 5,0 % en poids d'octisalate, 2,4 % en poids d'octocrylène ; et
un photostabilisant qui stabilise ledit agent d'écran solaire en présence dudit agent autobronzant pour permettre audit agent d'écran solaire de conserver une partie de ladite absorption de rayonnement d'UV caractéristique de celui-ci sur une plage de rayonnement UV, permettant ainsi audit agent d'écran solaire de rester photostable lorsqu'il est soumis audit rayonnement UV, ledit photostabilisant étant choisi dans le groupe constitué par les copolymères de bas poids moléculaires d'acide adipique et de néopentylglycol et ledit photostabilisant étant présent en une quantité de 1,0 % en poids ou 2,0 % en poids.

2. Composition cosmétique selon la revendication 1, comprenant en outre au moins un composant parmi un émollient, un humectant, un agent filmogène, un agent émulsifiant, un épaississant, un conservateur, un agent colorant, et un parfum.

3. Composition cosmétique selon la revendication 2, dans laquelle ledit émollient est choisi dans le groupe constitué par des glycérides de coco, de la cyclométhicone, de la diméthicone, du dicapryl-maléate, des triglycérides capryliques/capriques, du myristate d'isopropyle, du stéarate d'octyle, du linoléate d'isostéaryle, de l'huile de lanoline, de l'huile de coco, du beurre de cacao, du beurre de karité, de l'huile d'olive, de l'huile d'avocat, des extraits d'aloé, de l'huile de jojoba, de l'huile de ricin, des acides gras, des alcools gras, du diisopropyladipate, un ester d'hydroxybenzoate, des esters d'acide benzoïque d'alcools en C₉ à C₁₅, un iso-nonanoate d'isononyle, des alcanes, des silicones, des éthers, du benzoate d'alkyle en C₁₂ à C₁₅ et leurs combinaisons.

4. Composition cosmétique selon la revendication 3, dans laquelle ledit émollient est présent dans ladite composition cosmétique en une quantité de 0,25 % en poids à 4,0 % en poids.

5. Composition cosmétique selon la revendication 2, dans laquelle ledit humectant est choisi dans le groupe constitué par la glycérine, le pentylène glycol, l'hexylène glycol, le propylène glycol, le butylène glycol, le sorbitol, le PEG-4 et leurs combinaisons.

6. Composition cosmétique selon la revendication 5, dans laquelle ledit humectant est de la glycérine présente en une quantité de 1,0 % en poids à 10 % en poids.

7. Composition cosmétique selon la revendication 2, dans laquelle ledit agent filmogène est choisi dans le groupe constitué par des copolymères d'acrylates, des copolymères d'acrylates/acrylamides, des combinaisons de copolymères d'acrylates et d'alkylméthacrylate en C₁₂ à C₂₂, des polyéthylènes, des cires, des esters de polyvinyl-pyrrolidone (PVP)/diméthiconylacrylate/ polycarbamylpolyglycol, du PVP butylé, un copolymère de PVP/hexadécène, un copolymère de PVP/eicosène, du tricontanyl-PVP, du polyester-7 (et) du néopentylglycol diheptanolate et une combinaison de ceux-ci.

8. Composition cosmétique selon la revendication 7, dans laquelle ledit agent filmogène est présent en une quantité de 0,1 % en poids à 5,0 % en poids.

9. Composition cosmétique selon la revendication 2, dans laquelle ledit agent émulsifiant est un premier agent émulsifiant choisi dans le groupe constitué par les polymères croisés d'acrylate, l'acide polyacrylique, le méthacrylate de sodium, le polyacrylate de sodium, les polyacrylates, l'acide stéarique, les polysorbates, les éthers de stéaryle, l'alcool en C₁₄ à C₂₂ (et) l'alkyle en C₁₂ à C₂₀-glucoside et leurs combinaisons.

10. Composition cosmétique selon la revendication 9, dans laquelle ledit premier agent émulsifiant est présent en une quantité de 0,01 % en poids à 10 % en poids.

11. Composition cosmétique selon la revendication 9, comprenant en outre un deuxième agent émulsifiant choisi dans le groupe constitué par l'alcool de cétyle, l'alcool de stéaryle et leurs combinaisons.

12. Composition cosmétique selon la revendication 11, dans laquelle ledit deuxième agent émulsifiant est présent en une quantité de 0,01 % en poids à 5,0 % en poids.

13. Composition cosmétique selon la revendication 2, dans laquelle ledit épaississant est choisi dans le groupe constitué par une gomme synthétique et naturelle ou des produits de polymère, des agents épaississants polysaccharides, des épaississants associatifs, des modificateurs de rhéologie associatifs anioniques, des modificateurs de rhéologie associatifs non ioniques, des agents épaississants huileux, des polymères croisés d'acrylates/ alkylacrylate en C₁₀ à C₃₀, des copolymères d'acrylates/aminoacrylates/alkyle en C₁₀ à C₃₀ PEG-20 itaconate, des copolymères d'acrylate, des copolymères d'acrylates/stéareth-20 méthacrylate, des copolymères d'acrylates/ béhéneth-25 méthacrylate, des copolymères de PEG-150-alcool de décyle/SMDI, la PVP, le carbomère, un polymère croisé de PEG, des copolymères d'acrylates/palmeth-25 acrylates, des polysaccharides, des polyacrylates, le polyéther-1, des silicates de sodium magnésium, des carbomères de sodium, des polyacrylates de sodium, des polyméthacrylates de sodium, des polyacryloyldiméthyl-taurates de sodium, des copolymères d'acryloyldiméthyl-taurate de sodium, du carragheen de sodium, du carboxyméthyl-dextrane de sodium, de l'hydroxyéthylcellulose, de l'hydroxypropyl-cyclodextrane, des bentonites, de la trihydroxystéarine, de l'hydroxyde d'aluminium - magnésium stéarate, de la gomme xanthane et toutes combinaisons de ceux-ci.

14. Composition cosmétique selon la revendication 13, dans laquelle ledit épaississant est présent en une quantité de 0,01 % en poids à 10 % en poids.

15. Composition cosmétique selon la revendication 2, dans laquelle ledit conservateur est choisi dans le groupe constitué par la diazolidinyl-urée, l'iodopropynyl-butylcarbamate, la chlorométhylisothiazolinone, la méthylisothiazolinone, la vitamine E, les dérivés de vitamine E, l'acétate de vitamine E, la vitamine C, le BHT, le butylparabène, l'éthylparabène, le méthylparabène, le propylparabène, l'isobutylparabène, le phénoxyéthanol et leurs combinaisons.

16. Composition cosmétique selon la revendication 15, dans laquelle ledit conservateur est présent en une quantité de 0,01 % en poids à 2 % en poids.

17. Composition cosmétique selon la revendication 2, dans laquelle ledit agent colorant est choisi dans le groupe constitué par le caramel, la mélanine, un extrait botanique, l'oxyde de zinc, l'oxyde de fer, le dioxyde de titane et leurs combinaisons.

18. Composition cosmétique selon la revendication 1, comprenant en outre un conditionneur cutané choisi dans le groupe constitué par le caprylyl-glycol, la vaseline, la glycérine, l'aloé vera, l'allantoïne, le PCA sodium et leurs combinaisons.

19. Composition cosmétique selon la revendication 18, dans laquelle le conditionneur cutané est présent en une quantité allant jusqu'à 30 % en poids, de préférence, en une quantité de 0,1 % en poids à 10 % en poids et de manière davantage préférée, en une quantité de 0,25 % en poids à 5 % en poids.

20. Composition cosmétique selon la revendication 1, dans laquelle ladite partie de ladite absorption de rayonnement UV caractéristique est d'au moins 78 %, de préférence d'au moins 92 %.

21. Composition cosmétique selon la revendication 1, dans laquelle ladite plage de rayonnement UV est de 350 nm à 370 nm.

22. Composition cosmétique selon la revendication 1, la composition comprenant 5,0 % en poids de DHA, 1,0 % en poids d'acide adipique et d'un copolymère de néopentyl-glycol, 3,0 % en poids d'avobenzone, 5,0 % en poids d'homosalate, 5,0 % en poids d'octisalate, 2,4 % en poids d'octocrylène et jusqu'à 100 % en poids d'autres composants.

23. Composition cosmétique selon la revendication 1, la composition comprenant 5,0 % en poids de DHA, 2,0 % en poids d'acide adipique et d'un copolymère de néopentyl-glycol, 3,0 % en poids d'avobenzone, 5,0 % en poids d'homosalate, 5,0 % en poids d'octisalate, 2,4 % en poids d'octocrylène et jusqu'à 100 % en poids d'autres composants.
